# EUROPEAN PATENT SPECIFICATION

(11) **EP 3 068 356 B1**
(45) Date of publication and mention of the grant of the patent: **29.04.2020**
(21) Application number: 14796874.7
(22) Date of filing: 07.11.2014
(51) Int. Cl.: A61F 11/08, A61F 11/12

(54) **EARPIECE**
OHRSTÖPSEL
ÉCOUTEUR

(30) Priority: 13.11.2013 GB 201319986
(43) Date of publication of application: 21.09.2016
(73) Proprietor: Racal Acoustics Limited, Harrow, Middlesex HA1 4TR (GB)
(72) Inventor: HUSSEIN, Habib, Harrow Middlesex HA1 4TR (GB); DAVIDSON, Norman, Harrow Middlesex HA1 4TR (GB)
(74) Representative: Dolleymores
(86) International application number: PCT/GB2014/053325
(87) International publication number: WO 2015/071646

(56) References cited:
- EP-A1- 1 809 069
- US-A- 4 353 364
- US-A1- 2007 102 006

## Description

The present disclosure relates to an earpiece and to a headset comprising a pair of the earpieces, in particular a headset that is capable of providing hearing protection in environments with medium and/or high ambient noise.

In medium and/or high noise environments, hearing protection devices are often required which must include a communication means. Such hearing protection devices are normally provided in the form of circum-aural headsets or In-The-Ear (ITE) headsets.

When using a wired ITE headset a cable is used to connect the headset to the communications means or to headset electronics, which cable must be connected to the earpieces of the headset. Most ITE headsets are provided with earpieces that route the cable over the ear, since the cable then helps to maintain the headset in position during use. Routing the cable over the ear can, however, cause issues of cable retention, interference with glasses or eye protection, visual aids, etc. Accordingly, there may be situations where the user may prefer for the cable to be routed other than over the ear, particularly under or in front of the ear.

To date no ITE headset has been provided that provides sufficient hearing protection in environments having medium to high ambient noise levels and also allows a user to switch between wearing configurations with ease. The present invention arose in a bid to provide such a headset. The present inventors, through extensive research and experimentation, devised an earpiece for an ITE headset, and a headset comprising a pair of the earpieces, that allows for the cable to be routed over, under or in front of the ear, as desired, allowing flexibility of use and greater comfort.

Prior art earpieces are known from EP 1809069 and US 2007/0102006.

According to the present invention in a first aspect, there is provided an earpiece for a hearing protection device as recited by Claim 1.

The earbud may be constructed from foam, silicon or similar materials, or from a combination of materials, and is arranged to block the users ear canal to provide a means of passive sound attenuation. The earbud preferably comprises a concha retention means, which is arranged to engage a surface of the user's concha when the earbud is received by the ear canal of the user. The earbud may comprise a custom moulded earbud, which preferably substantially fills the concha of the user.

A spigot is preferably provided, which engages and supports the earbud, and the orientation and position of the spigot are preferably fixed.

The earbud comprises a longitudinal axis. When the earpiece is oriented for use, the longitudinal axis of the earbud preferably extends horizontally.

The angle between the cable extending from the body/the longitudinal axis of the cable entry and the longitudinal axis of the earbud in the vertical plane is preferably between 30 and 60 degrees. It may be any value within this range or may fall within any sub-range. It is most preferably substantially 45 degrees.

The angle between the cable extending from the body/the longitudinal axis of the cable entry and the longitudinal axis of the earbud in the horizontal plane is preferably between 70 and 100 degrees. It may be any value within this range or may fall within any sub-range. It is most preferably substantially 90 degrees.

The body may comprise a curved or substantially planar surface from which the earbud extends, wherein this surface substantially lies in a vertical plane in use.

The longitudinal axis of the cable entry extends at an oblique angles relative to the vertical and the horizontal planes of the body.

The earbud may lie in the horizontal plane and may be angled by between 15 and 45 degrees to the transverse horizontal axis. It may be angled by any value within this range or may fall within any sub-range. The angle is preferably 30 degrees.

The cable extending from the body/the longitudinal axis of the cable entry is preferably angled by between 15 and 35 degrees to the vertical plane (and the longitudinal horizontal axis of the body); and by between 30 and 60 degrees to the horizontal plane. It may be angled by any value within these ranges or may fall within any sub-ranges. The angles are most preferably substantially 25 and 45 degrees respectively.

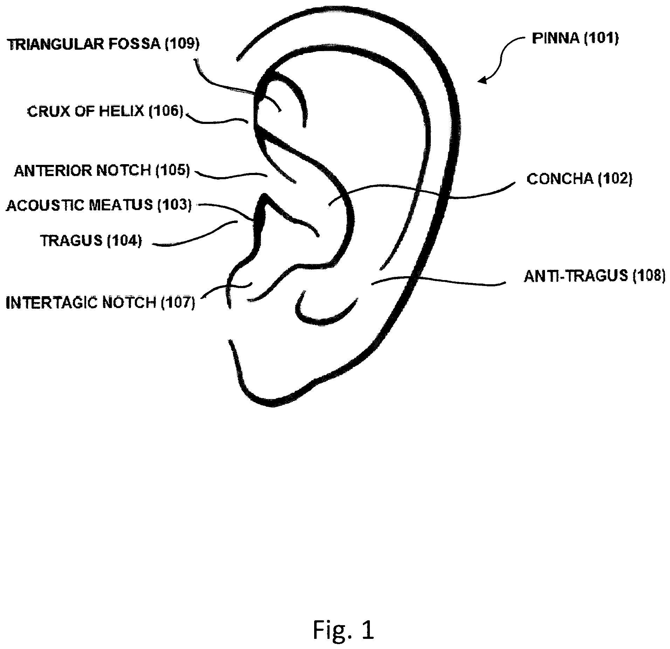

The pinna (101) is the visible part of the ear that resides outside of the head. The concha (102) is the bowl-shaped part of the pinna nearest the acoustic meatus (ear canal) (103). The tragus (104) is the small pointed eminence of the pinna, situated in front of the concha, and projecting backward over the meatus. The anterior notch (105) is a notch (above the tragus) that separates the tragus from the crus of the helix (106). The intertragic notch (107) is a notch (below the tragus) that separates the tragus from the antitragus (108). Figure 1 shows a labeled pinna, for reference.

A non-limiting embodiment will now be described, by way of example only, with reference to the accompanying drawings, in which:
Figure 1 shows a user's pinna with the parts thereof labeled;
Figures 2 and 3 show an ITE headset in accordance with the present invention, in use, in a user's right ear, with the cable routed over the ear;
Figures 4 and 5 show an ITE headset in accordance with the present invention, in use, in a user's right ear, with the cable routed under the ear;
Figures 6 and 7 show an ITE headset in accordance with the present invention, in use, in a user's right ear, with the cable routed in front of the ear;
Figure 8 shows a front end view of the body of the earpiece from the headset of Figures 2 to 7, oriented as if inserted in the left ear of a user with the cable routed over the ear;
Figure 9 shows the body of the earpiece from the headset of Figures 2 to 7 viewed from above, oriented as if inserted in the left ear of a user with the cable routed over the ear;and
Figure 10 shows a side view of the body of the earpiece from the headset of Figures 2 to 7, oriented as if inserted in the left ear of a user with the cable routed over the ear.

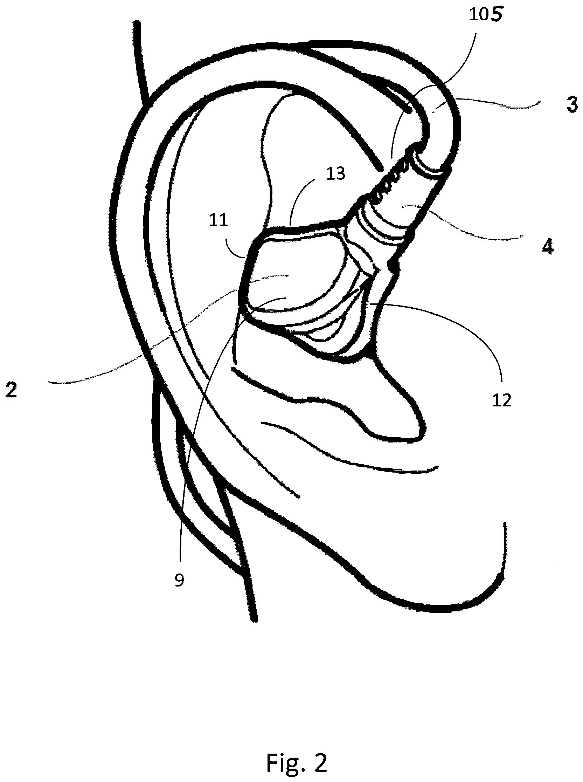

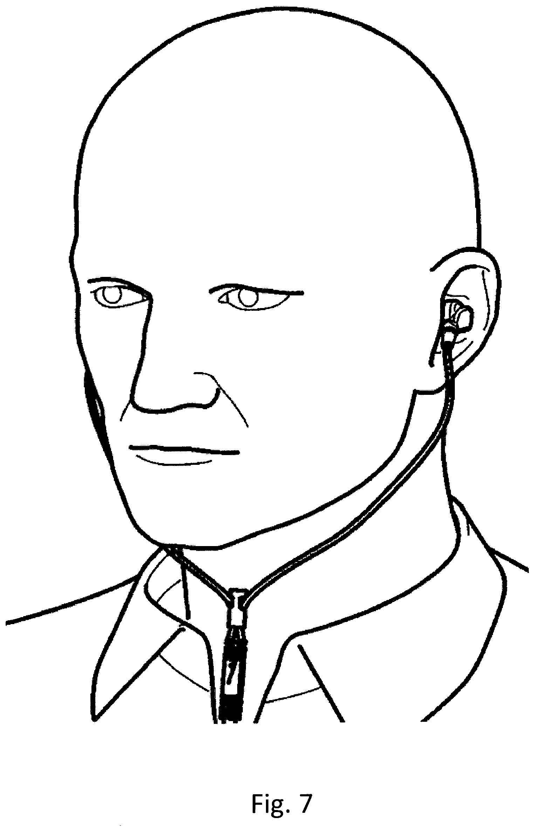

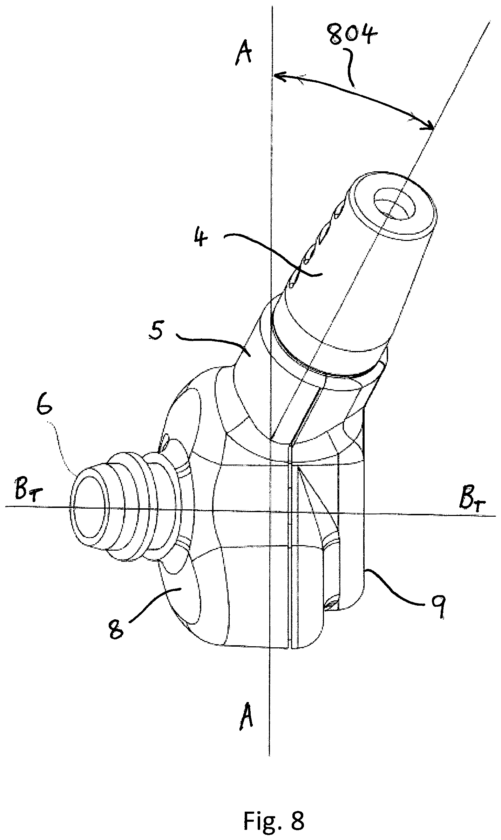

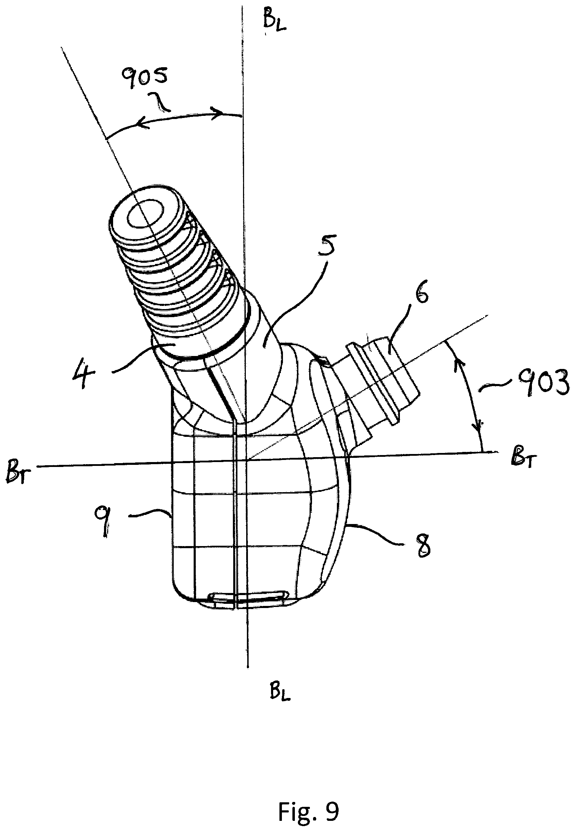

With reference to Figures 2 to 7, there is shown a headset, in accordance with the present invention, in use. The headset comprises a pair of earpieces (1). Only the earpiece in the right ear is visible in Figures 2 to 6 and only the earpiece in the left ear is visible in Figure 7, the other earpiece in each of the figures is obscured from view by the wearer's head. An earpiece of the headset of Figures 2 to 7 is shown from different angles in Figures 8 to 10. Note that the cable and earbud are omitted from Figures 8 to 10.

The headset in accordance with the present invention is arranged to provide suitable hearing protection to prevent a user suffering hearing damage in a medium or high ambient noise environment. Preferably, the passive attenuation of the headset is sufficient in itself to provide such suitable hearing protection.

A medium ambient noise environment is an environment where hearing damage or noise induced hearing loss can occur with long term exposure to the noise. The law of many countries attributes a continuous sound level of 85dbA to this environment. Hearing damage or noise induced hearing loss can occur after an exposure period of 8 hour per day in such an environment. For impulse or impact noise the level is set at 140db peak sound pressure level (SPL).

A high ambient noise environment is an environment where hearing damage or noise induced hearing loss can occur with short term exposure to the noise. The law of many countries attributes a continuous sound level of 105dbSPL to this environment. Hearing damage or noise induced hearing loss can occur after an exposure period of 1 hour per day.

The above definitions of medium and high ambient noise environments are adopted herein.

Each of the earpieces comprises a body (2), which houses a plurality of electrical components (described below) including at least one acoustic transducer, a cable (3), which carries signals to and/or from the electrical components in the body, and an earbud (not shown). The cable and the earbud are joined to the body and extend therefrom. At least the earbud is received by the wearer's pinna (101) during use, as shown, with the earbud received by and substantially filling the ear canal (103). Dependent on the form of earbud used, the earbud may additionally be received by the concha (102), as discussed below.

A cable entry (4), as shown, is provided to guide the cable to/from the body and provide support to the portion of the cable immediately outside the body. The cable entry may comprise a tapered sleeve that has its diameter gradually reduced along its axial length as it extends away from the body, as shown. The cable entry may, however, take numerous alternative forms, as will be readily appreciated by those skilled in the art. The cable entry is preferably formed from rubber, plastic or a similar material. The cable entry is preferably semi-rigid, such that it may support the cable at a desired angle from the body but may flex to be comfortably accommodated by the wearer's ear. The body may be provided with a protruding collar (5), as shown, which engages and supports the base of the cable entry.

Alternative means for guiding and supporting the wire as it exits the body may be provided, as will be appreciated by those skilled in the art. The present invention is not limited to the specific arrangement shown.

A spigot (6), as seen in Figures 8 to 10, is preferably provided as the means for attaching the earbud to the body. The spigot is preferably arranged to engage a hole in the earbud, which extends along the longitudinal axis of the earbud. The earbud may be attached to the spigot by interference fit or otherwise. The spigot and earbud will share a common longitudinal axis regardless of the specific attachment means. It should be noted that in alternative arrangements the spigot may be omitted with an alternative means of attaching the body and the earbud to one another being provided instead.

The earbud may take any conventional form and may be made from silicon, foam or similar. It is particularly preferred, however, that the earbud comprises concha retention means, which are arranged to engage a surface of the user's concha when the earbud is received by the ear canal of the user. The concha retention means may be of any conventional form, as will be appreciated by those skilled in the art. Typical concha retention means operate by hooking into the recess in the concha created by the crus of the helix (106), the triangular fossa (109) and rest of the concha (102). The earbud may be a custom moulded earbud. Such an earbud is made by taking a mould of the user's pinna and ear canal. A custom moulded earbud preferably substantially fills the concha of the user.

The earpiece as seen in Figures 2 to 10 and described herein has no moveable parts. The position and orientation of the spigot (6), and thereby the earbud, is fixed. The position and orientation of the collar (5) and the cable entry (4) are fixed, which fixes the position and orientation of the cable exiting the body.

The body may be formed, for example, from moulded plastic. The collar (5) and the spigot (6) are preferably integrally formed with the body by moulding.

The body comprises a first (inner) face (8) and a second (outer) face (9), which is opposed to the first face. The earbud (not shown) is connected to the first face. In the present arrangement, as discussed, a spigot (6) is provided, which extends from the first face and is arranged to engage the earbud. The first face is arranged to face inwardly in use (relative to a user's head) with the earbud extending therefrom for receipt by the ear canal of the user. The first face thus faces the (radially outwardly facing) surface of the concha in use. To provide a comfortable fit, the first surface is preferably curved. The spigot (and thereby the earbud) preferably extends from the first face at an oblique angle, as shown. Notably, however, and in contrast to prior art earpiece constructions, a longitudinal axis of the spigot (and thereby a longitudinal axis of the earbud) extends horizontally when the earpiece is oriented for use, as discussed in detail below.

The second face (9) is opposed to the first face and faces radially outwardly in use, as seen in Figures 2 to 7. A sidewall is arranged between the first and second faces. The sidewall extends between the first and second faces. The sidewall may be considered to form first and second end faces (11, 12), which are opposed to one another, and a pair of side faces (13, 14), which extend between the end faces and are opposed to one another.

The cable entry (4) (and collar (5)) preferably extends from a corner of the body where the outer face (9), second end face (12) and side face (13) meet, as shown. The cable entry (4) (and thereby the cable) extends outwards from the body at an oblique angle to all three faces in a direction away from the spigot (and thereby the earbud).

It should be appreciated that the body need not be formed in the shape described herein, it could, for example, be substantially spherical or otherwise formed, with the earbud and cable extending outwardly therefrom.

The earpieces of the headset are mirror images of one another in the present embodiment. The headset is configured, by the arrangement of the earpieces, so that the routing of the cables may be changed by swapping the earpieces between the ears of the wearer.

In use, as seen in Figures 2 to 7, the first end face (11) faces a rear face of the concha (102) and the second end face (12) faces the tragus (104). This is irrespective of the orientation of the earpiece during use. In contrast, each of the side faces (13, 14) may form an upper or a lower face of the earpiece depending on the orientation of the earpiece.

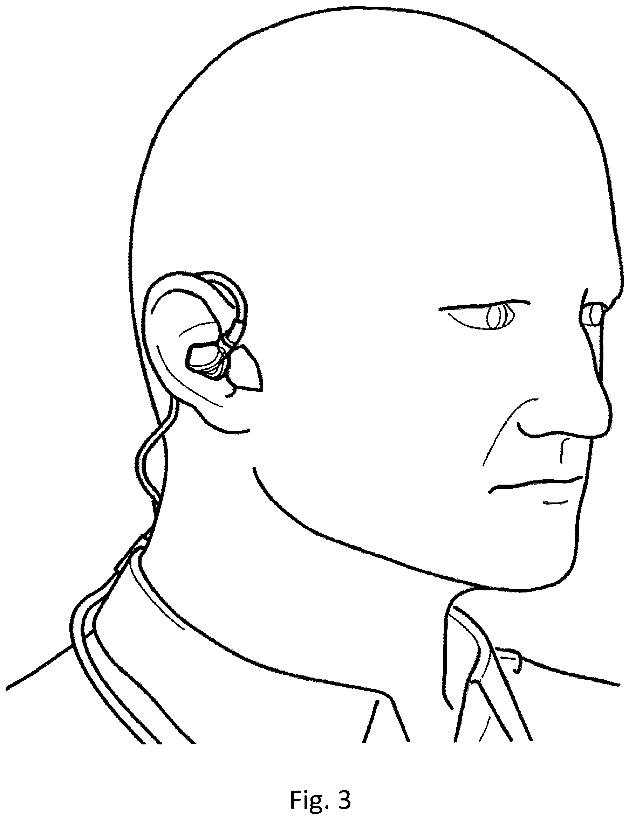

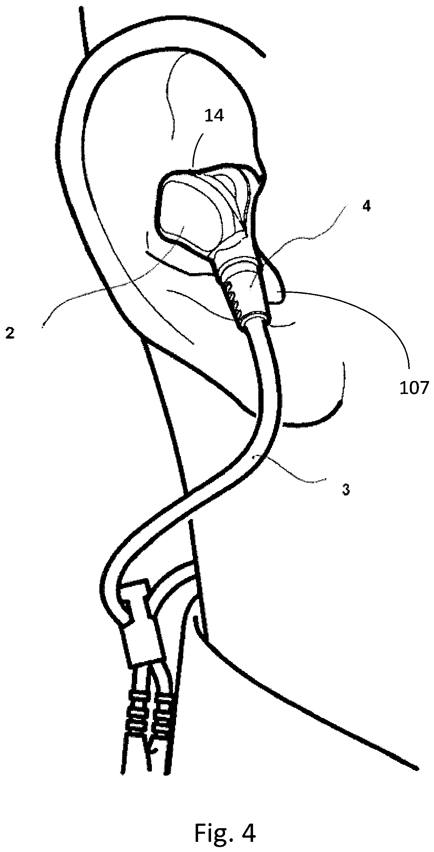

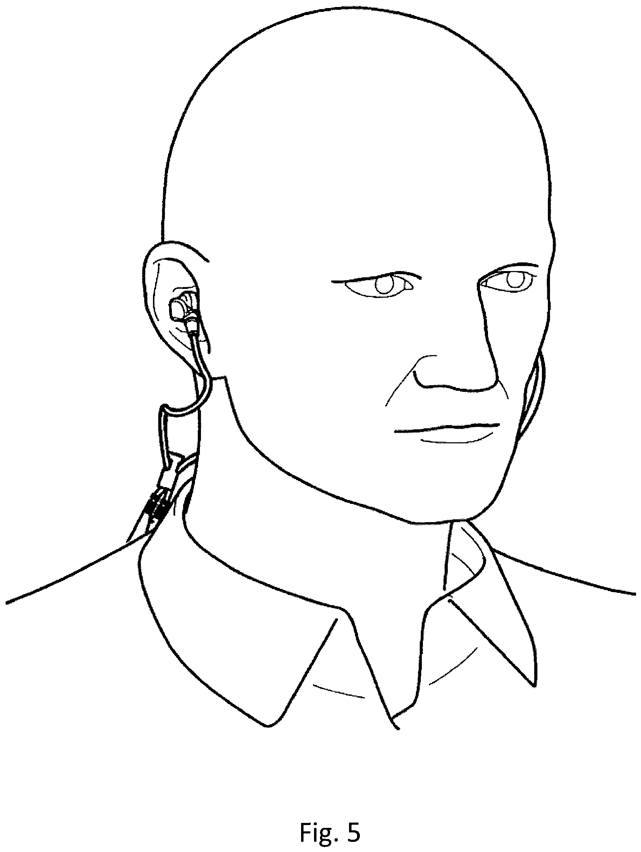

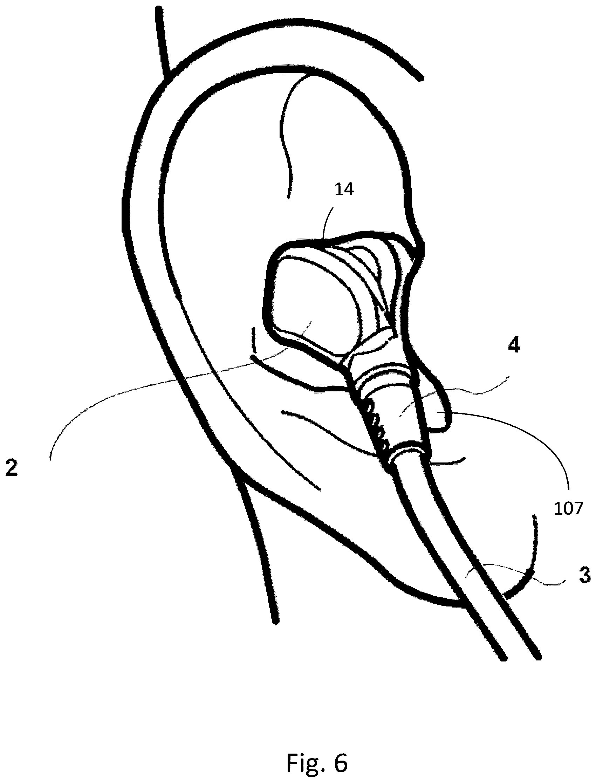

In Figures 2 and 3 the headset is worn in an over the ear configuration with the cable routed over the wearer's ear. In Figures 4 to 7 the configuration of the headset is changed with the cable routed under the ear (Figures 4 and 5) or in front of the ear (Figures 6 and 7). The change between the configurations of Figures 2 and 3 and Figures 4 to 7 is brought about by swapping the earpieces of the headset between the left and right ears of the wearer. In Figures 4 to 6 the earpiece shown in Figure 2 has been placed in the left ear with the other earpiece (the earpiece in the left ear in Figure 2) placed in the right ear.

When an earpiece of an ITE headset according to the present invention is fitted into the concha (102) and ear canal (acoustic meatus) (103) of an ear with the cable routed over the pinna (101), the cable entry (4) (and thereby the cable) is preferably located in the anterior notch (105), as shown in Figures 2 and 3.

When an earpiece of an ITE headset according to the present invention is fitted into the concha (102) and ear canal (acoustic meatus) (103) of an ear with the cable routed under the pinna (101), as shown in Figures 4 and 5, or with the cable routed in front of the pinna (101), as shown in Figures 6 and 7, the cable entry (4) (and thereby the cable) is preferably located in the intertragic notch (107).

The earpiece is configured such that in a first ear of a wearer, in a first orientation, with the earbud received by the ear canal of that ear, the cable entry extends upwardly and lies in the anterior notch of the first ear; and, in the other ear of the wearer, in a second orientation, in which the earpiece is rotated through substantially 180 degrees and with the earbud received by the ear canal of that other ear, the cable entry extends downwardly and lies in the intertragic notch of the other ear. The headset comprises a pair of the earpieces that are a mirror image of one another.

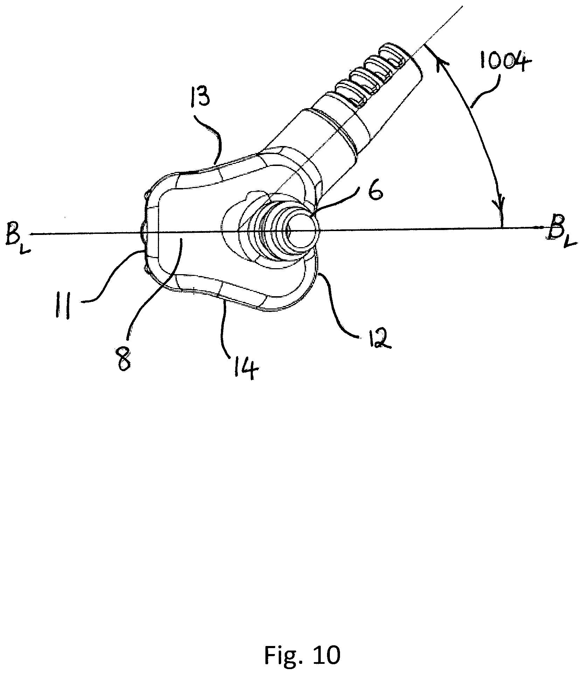

The spigot (6) (and thereby the earbud) has a longitudinal axis. A significant feature that aids in the swapping of the earpieces of a headset according to the present invention between ears, when the earbuds comprise concha retention means and/or are custom moulded earbuds, is the arrangement of the longitudinal axes of the spigots/earbuds such that they are horizontal. Figures 8 and 10 show an earpiece (minus the cable and earbud) oriented (with the cable extending upwardly) as if inserted in the left ear of a user. The line A-A represents a vertical axis of the body. The line B_{T}-B_{T} represents a transverse horizontal axis of the body and the line B_{L}-B_{L} represents a longitudinal horizontal axis of the body (which will be the front to back direction of the users head). The vertical axis and longitudinal horizontal axis both lie in a vertical plane. The horizontal axes both lie in a horizontal plane. The longitudinal axis of the spigot/earbud lies in the horizontal plane and at an oblique angle to the horizontal axes. The longitudinal axis of the spigot/earbud also lies perpendicular to the vertical axis A-A and thereby the vertical plane, as shown, such that the longitudinal axis of the spigot /earbud is horizontal when the earpiece is oriented for use.

The spigot (6)/earbud is angled (903) in a single plane (the horizontal plane) to present an attached earbud for insertion into the ear canal. In the example, the spigot (6) may be angled (903) by substantially 30 degrees from the transverse horizontal axis (B_{T}-B_{T}). The angle may be any angle between 15 and 45 degrees.

The cable entry (4) has a longitudinal axis. The longitudinal axis of the cable entry lies at an oblique angle relative to the vertical axis A-A and to the horizontal axis B-B, and thereby to the vertical and horizontal planes.

The cable entry (4) is preferably angled (804, 905, 1004) in two planes, as shown. In the example, the cable entry (4) is angled by substantially 25 degrees (804, 905) to the vertical plane (and the longitudinal horizontal axis of the body); and by substantially 45 degrees (1004) to the horizontal plane. These angles may, for example, be any angles between 15 and 35 degrees and between 30 and 60 degrees respectively.

The angle between the cable entry (4) and the longitudinal axis of the earbud/spigot in the vertical plane may be between 30 and 60 degrees. In the present arrangement it is substantially 45 degrees.

The angle between the cable entry (4) and the longitudinal axis of the earbud/spigot in the horizontal plane may be between 70 and 100 degrees. In the present arrangement it is substantially 90 degrees.

Pinnas and ear canals of the users of the invention will vary from user to user. The angles of the spigot and cable entry detailed above are determined as being able to fit the majority of users. The spigot and cable entry in the described example are fixed in relation to the main body of the earpiece. In alternative arrangements, by using a spigot and cable entry with some movement in relation to the main body the earpiece may be adapted to fit a greater percentage of users. Moreover, whilst the angles/ranges detailed above are preferred, the angles may be varied in alternative arrangements, as will be readily appreciated by those skilled in the art.

Conventional noise reduction components/circuitry may be introduced into the earpiece, as will be readily appreciated by those skilled in the art. Such components/circuitry may comprise a feed forward circuit and a speaker (acoustic transducer) in addition to a microphone. The signal of the noise appearing at the user's ear which is detected by the microphone is inverted and added to the drive signal of the speaker, creating a cancellation signal.

A "talk-through" function may be provided by the introduction of a talk-through circuit into the earpiece, which talk-through circuit passes an electrical signal from the microphone on to the speaker and thus onto the user's hearing.

The noise cancelling and talk through functions may be provided in combination with one another or independently of one another.

Various other electrical circuitry and transducers may be introduced into any earpiece in accordance with the present invention, as will be readily appreciated by those skilled in the art.

Numerous alternatives and modifications within the scope of the appended claims are possible, as will be readily appreciated by those skilled in the art.

## Claims

1. An earpiece (1) for a hearing protection device arranged to provide hearing protection in environments with medium to high ambient noise levels, the earpiece comprising a body (2), an earbud, which is arranged to be inserted into an ear canal of a user to block the ear canal of the user, a cable (3), and a cable entry (4), which extends from the body and through which the cable passes,
wherein the earbud and the cable entry extend from the body,
wherein the body, in use, comprises a vertical axis (A-A) and transverse and longitudinal horizontal axes (B_{T}-B_{T}, B_{L}-B_{L}), the vertical axis lying in a vertical plane, the longitudinal horizontal axis lying in the vertical plane and in a horizontal plane and the transverse horizontal axis lying in the horizontal plane, and
wherein the longitudinal axis of the earbud extends at an oblique angle to the transverse horizontal axis of the body,
wherein the earpiece is arranged such that when the earbud is received by the ear canal of the user's ear, the longitudinal axis of the body extends in the front to back direction of the user's head,
wherein the earpiece is arranged such that when the earbud is received by the ear canal of the user's left ear, the cable entry extends from the body in an upwardly direction to lie in the anterior notch or in a downwardly direction to lie in the intertragic notch, and when the earbud is received by the ear canal of the user's right ear, the cable entry extends from the body in the other of the upwardly or downwardly directions to lie in the other of the anterior notch or the intertragic notch, and
wherein a longitudinal axis of the cable entry lies at oblique angles relative to the vertical and horizontal planes of the body.

2. An earpiece as claimed in Claim 1, wherein the earbud comprises a concha retention means, which is arranged to engage a surface of the user's concha when the earbud is received by the ear canal of the user.

3. An earpiece as claimed in any preceding claim, wherein the earbud comprises a custom moulded earbud.

4. An earpiece as claimed in Claim 3, wherein the earbud is arranged to substantially fils the concha of the user.

5. An earpiece as claimed in any preceding claim, wherein the orientation and position of the earbud are fixed.

6. An earpiece as claimed in any of Claims 3 to 5, wherein the orientation and position of the cable entry are fixed.

7. An earpiece as claimed in any preceding claim, wherein the angle between a longitudinal axis of the cable entry and the longitudinal axis of the earbud in the vertical plane is between 30 and 60 degrees.

8. An earpiece as claimed in any preceding claim, wherein the angle between a longitudinal axis of the cable entry and the longitudinal axis of the earbud in the vertical plane is substantially 45 degrees.

9. An earpiece as claimed in any preceding claim, wherein the angle between a longitudinal axis of the cable entry and the longitudinal axis of the earbud in the horizontal plane is between 70 and 100 degrees.

10. An earpiece as claimed in any preceding claim, wherein the angle between a longitudinal axis of the cable entry and the longitudinal axis of the earbud in the horizontal plane is substantially 90 degrees.

11. An earpiece as claimed in any preceding claim, wherein the longitudinal axis of the earbud lies in the horizontal plane of the body.

12. An earpiece as claimed in any preceding claim, wherein the longitudinal axis of the earbud extends at an angle of 15 to 45 degrees to the transverse horizontal axis, and preferably at an angle of substantially 30 degrees to the transverse horizontal axis.

13. An earpiece as claimed in any preceding claim, wherein the angle relative to the vertical plane, or the longitudinal horizontal axis, is between 15 and 35 degrees, and preferably substantially 25 degrees.

14. An earpiece as claimed in any preceding claim, wherein the angle relative to the horizontal plane is between 30 and 60 degrees, and preferably substantially 45 degrees.

15. A headset comprising a pair of earpieces as claimed in any preceding claim.

## Patentansprüche

1. Ohrstecker (1) für eine Gehörschutzvorrichtung, die zur Bereitstellung von Gehörschutz in Umgebungen mit mittleren bis hohen Umgebungsgeräuschpegeln angeordnet ist, wobei der Ohrstecker Folgendes umfasst: einen Körper (2), einen Ohrstöpsel, der zur Einführung in einen Gehörgang eines Anwenders angeordnet ist, um den Gehörgang des Anwenders zu blockieren, ein Kabel (3) und eine Kabeleinführung (4), die sich von dem Körper erstreckt und durch die das Kabel hindurchgeht,
wobei sich der Ohrstöpsel und die Kabeleinführung von dem Körper erstrecken, wobei der Körper bei Verwendung eine vertikale Achse (A-A) und eine querlaufende und eine längsgerichtete horizontale Achse (B_{T}-B_{T}, B_{L}-B_{L}) umfasst, wobei die vertikale Achse in einer vertikalen Ebene liegt, die längsgerichtete horizontale Achse in der vertikalen Ebene und in einer horizontalen Ebene liegt und die querlaufende horizontale Achse in der horizontalen Ebene liegt, und
wobei sich die längsgerichtete Achse des Ohrstöpsels in einem schrägen Winkel zu der querlaufenden horizontalen Achse des Körpers erstreckt,
wobei der Ohrstecker derart angeordnet ist, dass, wenn der Ohrstöpsel von dem Gehörgang des Ohrs des Anwenders aufgenommen wird, sich die längsgerichtete Achse des Körpers in der vorderen zur hinteren Richtung des Kopfes des Anwenders erstreckt,
wobei der Ohrstecker derart angeordnet ist, dass, wenn der Ohrstöpsel von dem Gehörgang des linken Ohrs des Anwenders aufgenommen wird, sich die Kabeleinführung von dem Körper in einer nach oben gerichteten Richtung erstreckt, um in der anterioren Kerbe zu liegen, oder in einer nach unten gerichteten Richtung erstreckt, um in der intertragischen Kerbe zu liegen, und, wenn der Ohrstöpsel von dem Gehörgang des rechten Ohrs des Anwenders aufgenommen wird, sich die Kabeleinführung von dem Körper in der anderen der nach oben gerichteten oder nach unten gerichteten Richtung erstreckt, um in der anderen der anterioren Kerbe oder der intertragischen Kerbe zu liegen, und
wobei eine längsgerichtete Achse der Kabeleinführung in schrägen Winkeln in Bezug auf die vertikale und horizontale Ebene des Körpers liegt.

2. Ohrstecker nach Anspruch 1, wobei der Ohrstöpsel ein Concha-Haltemittel umfasst, das zum Eingreifen in eine Oberfläche der Concha des Anwenders angeordnet ist, wenn der Ohrstöpsel von dem Gehörgang des Anwenders aufgenommen wird.

3. Ohrstecker nach einem vorstehenden Anspruch, wobei der Ohrstöpsel einen kundenspezifisch geformten Ohrstöpsel umfasst.

4. Ohrstecker nach Anspruch 3, wobei der Ohrstöpsel angeordnet ist, um im Wesentlichen die Concha des Anwenders auszufüllen.

5. Ohrstecker nach einem vorstehenden Anspruch, wobei die Ausrichtung und Position des Ohrstöpsels fixiert sind.

6. Ohrstecker nach einem der Ansprüche 3 bis 5, wobei die Ausrichtung und Position der Kabeleinführung fixiert sind.

7. Ohrstecker nach einem vorstehenden Anspruch, wobei der Winkel zwischen einer längsgerichteten Achse der Kabeleinführung und der längsgerichteten Achse des Ohrstöpsels in der vertikalen Ebene zwischen 30 und 60 Grad beträgt.

8. Ohrstecker nach einem vorstehenden Anspruch, wobei der Winkel zwischen einer längsgerichteten Achse der Kabeleinführung und der längsgerichteten Achse des Ohrstöpsels in der vertikalen Ebene im Wesentlichen 45 Grad beträgt.

9. Ohrstecker nach einem vorstehenden Anspruch, wobei der Winkel zwischen einer längsgerichteten Achse der Kabeleinführung und der längsgerichteten Achse des Ohrstöpsels in der horizontalen Ebene zwischen 70 und 100 Grad beträgt.

10. Ohrstecker nach einem vorstehenden Anspruch, wobei der Winkel zwischen einer längsgerichteten Achse der Kabeleinführung und der längsgerichteten Achse des Ohrstöpsels in der horizontalen Ebene im Wesentlichen 90 Grad beträgt.

11. Ohrstecker nach einem vorstehenden Anspruch, wobei die längsgerichtete Achse des Ohrstöpsels in der horizontalen Ebene des Körpers liegt.

12. Ohrstecker nach einem vorstehenden Anspruch, wobei sich die längsgerichtete Achse des Ohrstöpsels in einem Winkel von 15 bis 45 Grad zu der querlaufenden horizontalen Achse und bevorzugt in einem Winkel von im Wesentlichen 30 Grad zu der querlaufenden horizontalen Achse erstreckt.

13. Ohrstecker nach einem vorstehenden Anspruch, wobei der Winkel in Bezug auf die vertikale Ebene oder die längsgerichtete horizontale Achse zwischen 15 und 35 Grad und bevorzugt im Wesentlichen 25 Grad beträgt.

14. Ohrstecker nach einem vorstehenden Anspruch, wobei der Winkel in Bezug auf die horizontale Ebene zwischen 30 und 60 Grad und bevorzugt im Wesentlichen 45 Grad beträgt.

15. Headset, das ein Paar Ohrstecker nach einem vorstehenden Anspruch umfasst.

## Revendications

1. Ecouteur (1) pour un dispositif de protection de l'ouïe arrangé pour fournir une protection de l'ouïe dans des environnements à niveaux de bruit ambiant de moyens à élevés, l'écouteur comprenant un corps (2), un embout auriculaire, qui est arrangé pour être introduit dans un conduit auditif d'un utilisateur pour bloquer le conduit auditif de l'utilisateur, un câble (3), et une entrée de câble (4), qui s'étend du corps et à travers laquelle passe le câble,
dans lequel l'embout auriculaire et l'entrée de câble s'étendent du corps,
dans lequel le corps, en emploi, comprend un axe vertical (A-A) et des axes horizontaux tranversal et longitudinal (B_{T}-B_{T}, B_{L}-B_{L}), l'axe vertical se trouvant dans un plan vertical, l'axe horizontal longitudinal se trouvant dans le plan vertical et dans un plan horizontal et l'axe horizontal transversal se trouvant dans le plan horizontal, et
dans lequel l'axe longitudinal de l'embout auriculaire s'étend à un angle oblique à l'axe horizontal transversal du corps,
dans lequel l'écouteur est arrangé de telle sorte que lorsque l'embout auriculaire est reçu par le conduit auditif de l'oreille de l'utilisateur, l'axe longitudinal du corps s'étend dans la direction d'avant en arrière de la tête de l'utilisateur,
dans lequel l'écouteur est arrangé de telle sorte que lorsque l'embout auriculaire est reçu par le conduit auditif de l'oreille gauche de l'utilisateur, l'entrée de câble s'étend du corps dans une direction vers le haut pour se trouver dans l'échancrure antérieure ou bien dans une direction vers le bas pour se trouver dans l'échancrure intertragienne, et lorsque l'embout auriculaire est reçu par le conduit auditif de l'oreille droite de l'utilisateur, l'entrée de câble s'étend du corps dans l'autre d'entre la direction vers le haut ou la direction vers le bas pour se trouver dans l'autre d'entre l'échancrure antérieure ou l'échancrure intertragienne, et
dans lequel un axe longitudinal de l'entrée de câble se trouve à des angles obliques par rapport aux plans vertical et horizontal du corps.

2. Ecouteur selon la revendication 1, dans lequel l'embout auriculaire comprend un moyen de rétention dans la conque, qui est arrangé pour engager une surface de la conque de l'utilisateur lorsque l'embout auriculaire est reçu par le conduit auditif de l'utilisateur.

3. Ecouteur selon l'une quelconque des revendications précédentes, dans lequel l'embout auriculaire comprend un embout auriculaire moulé sur mesure.

4. Ecouteur selon la revendication 3, dans lequel l'embout auriculaire est arrangé pour remplir sensiblement la conque de l'utilisateur.

5. Ecouteur selon l'une quelconque des revendications précédentes, dans lequel l'orientation et la position de l'embout auriculaire sont fixes.

6. Ecouteur selon l'une quelconque des revendications 3 à 5, dans lequel l'orientation et la position de l'entrée de câble sont fixes.

7. Ecouteur selon l'une quelconque des revendications précédentes, dans lequel l'angle entre un axe longitudinal de l'entrée de câble et l'axe longitudinal de l'embout auriculaire dans le plan vertical est d'entre 30 et 60 degrés.

8. Ecouteur selon l'une quelconque des revendications précédentes, dans lequel l'angle entre un axe longitudinal de l'entrée de câble et l'axe longitudinal de l'embout auriculaire dans le plan vertical est sensiblement de 45 degrés.

9. Ecouteur selon l'une quelconque des revendications précédentes, dans lequel l'angle entre un axe longitudinal de l'entrée de câble et l'axe longitudinal de l'embout auriculaire dans le plan horizontal est d'entre 70 et 100 degrés.

10. Ecouteur selon l'une quelconque des revendications précédentes, dans lequel l'angle entre un axe longitudinal de l'entrée de câble et l'axe longitudinal de l'embout auriculaire dans le plan horizontal est sensiblement de 90 degrés.

11. Ecouteur selon l'une quelconque des revendications précédentes, dans lequel l'axe longitudinal de l'embout auriculaire se trouve dans le plan horizontal du corps.

12. Ecouteur selon l'une quelconque des revendications précédentes, dans lequel l'axe longitudinal de l'embout auriculaire s'étend à un angle de 15 à 45 degrés à l'axe horizontal transversal, et de préférence à un angle sensiblement de 30 degrés à l'axe horizontal transversal.

13. Ecouteur selon l'une quelconque des revendications précédentes, dans lequel l'angle par rapport au plan vertical, ou l'angle horizontal longitudinal, est d'entre 15 et 35 degrés, et de préférence sensiblement de 25 degrés.

14. Ecouteur selon l'une quelconque des revendications précédentes, dans lequel l'angle par rapport au plan horizontal est d'entre 30 et 60 degrés, et de préférence sensiblement de 45 degrés.

15. Casque d'écoute comprenant une paire d'écouteurs selon l'une quelconque des revendications précédentes.
